**Europäisches Patentamt**

**European Patent Office**

**Office européen des brevets**

(19)

(11) Veröffentlichungsnummer: **0 154 872**

**B1**

(12) **EUROPÄISCHE PATENTSCHRIFT**

(45) Veröffentlichungstag der Patentschrift: **22.11.90**

(51) Int. Cl.⁵: **A 61 K 35/78**

(21) Anmeldenummer: **85102014.9**

(22) Anmeldetag: **23.02.85**

(54) Verfahren zur Herstellung flavonreicher Kamillenextrakte.

(30) Priorität: **16.03.84 DE 3409619**

(43) Veröffentlichungstag der Anmeldung:
**18.09.85 Patentblatt 85/38**

(45) Bekanntmachung des Hinweises auf die
Patenterteilung:
**22.11.90 Patentblatt 90/47**

(84) Benannte Vertragsstaaten:
**AT BE CH DE FR GB IT LI SE**

(56) Entgegenhaltungen:
**EP-A-0 175 185**
**DE-A-2 402 802**
**DE-A-3 423 207**
**FR-A-1 015 120**

(73) Patentinhaber: **ASTA Pharma Aktiengesellschaft**
**Weismüllerstrasse 45**
**D-6000 Frankfurt am Main 1 (DE)**

(72) Erfinder: **Isaac, Otto, Dr.**
**Liesingstrasse 8**
**D-6450 Hanau 9 (DE)**
Erfinder: **Carle, Reinhold, Dr.**
**Im Taubhaus 56a**
**D-6074 Rödermark (DE)**
Erfinder: **Dölle, Bernd, Dr.**
**Steubenstrasse 133**
**D-6070 Langen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Zur Herstellung von Kamillenextrakten wird üblicherweise eine Ganzdroge verwendet. Diese besteht aus den getrockneten Blütenköpfchen einschliesslich der zugehörigen Stielreste. Die Blütenköpfchen bestehen aus Blütenboden mit Hüllkelch, 12—20 Zungen- (Strahlen-) blüten und zahlreichen Röhrenblüten. Der Anteil der Zungenblüten an den Ganzdroge liegt bei etwa 5—6%, der Röhrenblütenanteil bei 67—69% und der Rest entfällt auf Blütenböden mit Hüllkelch und Blütenstiel.

Je nach Reifezustand der Blütenköpfchen bei der Ernte, Art der Trocknung, Verarbeitung, Verpackung und Lagerung der Droge zerfällt ein Teil der Ganzdroge in die einzelnen Bestandteile, wobei der hauptsächlich aus Röhrenblüten bestehende Kamillengrus und die vorzugsweise aus Zungenblüten bestehenden Flügelblätter anfallen. Diese Flügelblätter und dieser Kamillengrus werden im allgemeinen bereits bei der Drogengewinnung abgesiebt.

Der Kamillengrus und die Flügelblätter finden im Gemisch mit Kamillenfeinschnitt (das ist gehäckseltes Kamillenkraut) Verwendung als Füllung von Teeaufgussbeuteln. Kamillengrus wird ausserdem auch zur Gewinnung von Saatgut verwendet.

Für die Herstellung von Kamillenextrakten wird überlicherweise die von Grus und Flügelblättern weitgehend befreite Kamillenganzdroge eingesetzt. In einer solchen Ganzdroge ist jedoch das wichtige Flavon Apigenin und dessen Glykoside nicht mehr oder nur noch in geringem Masse vorhanden, da diese Inhaltsstoffe hauptsächlich in den Flügelblättern vorkommen. Aufgrund der ausgeprägten muskulotrop spasomolytischen Wirkung von Apigenin und der ebenfalls spasmolytisch wirksamen Apigeninglykoside sind Extrakte mit einem hohen Gehalt an diesen Flavonen von therapeutischem Interesse.

Da diese Flügeblätter einen weitaus geringeren Gehalt an ätherischem Öl haben als die Röhrenblüten beziehungsweise die intakten Blütenköpfchen, wurden sie bis jetzt nicht für eine Extraktion der Kamille mit dem Ziel der Herstellung von Kamillenextrakten verwendet.

Überraschenderweise wurde nun gefunden, daß bei der Extraktion mit den üblicherweise zur Kamillenextraktion verwendeten Lösungsmitteln ein Teil der Blütenköpfchen beziehungsweise der bisher verwendeten Ganzdroge durch Flügelblätter ersetzt werden kann, ohne dass der Gehalt an ätherischem Öl und seinen Bestandteilen zurückgeht. Man erhält dadurch Kamillenextrakte mit einem erhöhten Flavongehalt von mindestens 80 μm % an natürlichen Flavonen (bezogen auf den Extrakt) (insbesondere Apigenin und seine Glykoside) bei praktisch unverändertem Gehalt an ätherischem Öl die durch Extraktion einer Kamillendroge und Kamillen flügel blättern erhalten werden.

Für das erfindungsgemässe Verfahren wird eine wie oben beschriebene Kamillenganzdroge (ohne Grus- und Flügelblattanteil) oder eine übliche Kamillendroge, bei der die teilweise abgefallenen Röhrenblüten und Flügelblätter nicht entfernt sind, eingesetzt. Die zu extrahierende Kamille (beispielsweise Kamillenblüten, alle Sorten der Art Matricaria recutita) soll möglichst nicht länger als ein Jahr unter üblichen Lagerungsbedingungen gelagert sein, da sonst ein zu starker Verlust an ätherischem Öl und dessen Bestandteilen eintritt. Die Kamille soll auf übliche Weise getrocknet worden sein, beispielsweise durch künstliche Luftzufuhr oder auch durch Trocknung im Sonnenlicht (Temperatur beispielsweise 30—70°C, gegebenenfalls auch bis 100°C). Vorzugsweise soll die Trocknung sofort nach der Ernte und in dünnen Schichten (beispielsweise 5—20 cm Dicke) bei einer Lufttemperatur von nicht höher als 50°C erfolgen unter Vermeidung von direktem Sonnenlicht. Im allgemeinen beträgt der Feuchtigkeitsgehalt der einzusetzenden Kamillendroge 3—15%, insbesondere 8—10%.

Insbesondere ist für das erfindungsgemäße Verfahren eine Kamillendroge (Extraktionsware) geeignet mit einem Gehalt an ätherischem Öl von mindestens 750 mg%, beispielsweise 750 bis 1200 mg%, einem Anzulengehalt von mindestens 50 mg%, beispielsweise 50 bis 250 mg% und einem Bisabololgehalt von mindestens 150 mg%, beispielsweise 150 bis 300 mg%. Bei der Kamille kann es sich um diploide oder tetraploide Kamille handeln. Vorzugsweise eignet sich für das erfindungsgemässe Verfahren eine Kamillendroge gemäß der deutschen Patentschrift 24 02 802 sowie den deutschen Patentanmeldungen P 34 23 207 und P 34 46 216.

Die verwendeten Flügeblätter können von der gleichen Kamille stammen, aus der die Droge hergestellt wurde. Selbstverständlich können die Flügelblätter aber auch von einer Kamille anderer Provenienz stammen.

Die Kamillenflügelblätter können frisch oder getrocknet eingesetzt werden. Unter frischen Kamillenflügelblättern werden solche verstanden, die innerhalb von 24 Stunden nach dem Pflücken extrahiert werden oder innerhalb dieser Zeit eingefroren werden.

Der Anteil der Flügelblätter, welcher der Kamillendroge zugesetzt wird, liegt beispielsweise zwischen 5 und 30, vorzugsweise 10 bis 20 Gewichtsteilen der getrockneten Flügelblätter bezogen auf 100 Gewichtsteile des eingesetzten Drogenmaterials (Droge mit üblichem Flügelblattanteil von 5 bis 6 Gewichtsprozent oder Droge, bei welcher die Flügelblätter (Zungenblüten) durch Absieben entfernt wurden).

Das Verhältnis der zugesetzten getrockneten Flügelblätter zu den getrockneten Kamillenblütenköpfchen (Röhrenblüten und Blütenboden mit Hüllkelch und Blütenstielanteil) beträgt zum Beispiel: 1 zu 9 bis 1 zu 2, 3, vorzugsweise 1 zu 4 Gewichtsteile. Falls die Flügelblätter in Form von frischen Flügelblättern verwendet werden, ist eine entsprechend höhere Gewichtsmenge an Flügelblättern zu verwenden (das heißt, die Gewichtsmenge, die dem Drogenäquivalent entspricht). Im allgemeinen wird man bei frischen Flügelblättern das 3—5 fache, insbesondere 4—5 fache der Gewichtsmenge nehmen, die im Falle von getrockneten Flügelblättern eingesetzt wird.

Die Flügelblätter (Zungenblüten) enthalten nur etwa 12% des in den Kamillenblüten enthaltenen ätherischen Öls.

Die Kamillenblüten und die Flügelblätter können zerkleinert verwendet werden. Bei den Blüten ist es jedoch günstiger, wenn diese möglichst unzerkleinert sind. Die Flügelblätter können vor der Extraktion homogen mit der Kamillendroge vermischt werden. Falls die Extraktion unter Verwendung vom Mischmaschinen erfolgt, entsteht eine homogene Vermischung der Flügelblätter mit der eingesetzten Droge im Verlauf der Extraktion; eine vorherige Vermischung von Flügelblättern und Droge ist dann nicht erforderlich. Es ist auch möglich, die Kamillendroge und die Flügelblätter getrennt mit den angegebenen Mitteln zu extrahieren und dann die Extrakte zu vereinigen, wobei der Flügelblattextrakt zweckmäßig unter schonenden Bedingungen (zum Beispiel Vakuum) eingeengt wird.

Zur Durchführung des erfindungsgemässen Verfahrens können Mischeinrichtungen, zum Beispiel sogenannte Muldenmischmaschinen, Perkolatoren und andere geeignete Extraktionsapparaturen verwendet werden. Die Temperatur während der Extraktion beträgt beispielsweise 10 bis 50°C. Eine Kühlung ist nicht erforderlich.

Falls die Extraktion mittels Mischmaschinen erfolgt, soll die Drehzahl des Mischwerkes insbesondere so eingestellt werden, daß jeweils die Umlaufgeschwindigkeit eines Punktes an der Peripherie des Mischwerkes zwischen 2,8 und 1,4 m/Sekunde liegt, wobei die Extraktionszeit vorzugsweise zwischen 15 Minuten und 3 Stunden, insbesondere 30 bis 120 Minuten liegt.

Für ein Mischwerk mit einem Radius von 55 cm ist zum Beispiel eine Umdrehungsgeschwindigkeit von 50 bis 250 Umdrehungen/Minute günstig, wobei die Extraktionszeit dann beispielsweise 15 Minuten bis 3 Stunden beträgt. Vorzugsweise wählt man bei einem Mischer dieser Größe eine Umdrehungsgeschwindigkeit von 50 bis 250 Umdrehungen/Minute, wobei eine Extraktionsziet von 15 bis 120 Minuten ausreichend ist.

Als Lösungsmittel kommen insbesondere gerade oder verzweigte aliphatische, ein- oder mehrwertige Alkohole mit 1 bis 6 Kohlenstoffatomen in Frage, wie zum Beispiel Methanol, Ethanol, Propanol-(2), Butanol, Glycerin, Solketal (2-Dimethyl-4-oxymethyl-1,3-dioxolan) und ähnliche, sowie Gemische dieser Lösungsmittel mit Wasser.

Es können auch Gemische dieser Lösungsmittel verwendet werden. Die Mindestmenge an Lösungsmitteln beträgt 2 Teile Lösungsmittel auf 1 Teil Droge. Im allgemeinen verwendet man 2 bis 20 Teile Lösungsmittel auf 1 Teil Droge, insbesondere 3 bis 10 Teile Lösungsmittel auf 1 Teil Droge.

Die erfindungsgemäss erhaltenen Kamillenextrakte enthalten mindestens 80, vorzugsweise 90—170 mg% an natürlichen Flavonen, wobei es sich hierbei im wesentlichen um Apigenin und Apigeninglukoside (berechnet als Apigenin) handelt.

Der Gehalt an ätherischem Öl der erfindungsgemässen Kamillenextrakte ist mindestens 80, vorzugsweise 120—150 mg%.

Der Gehalt an natürlichen Flavonen (Apigenin und dessen Glukosiden) in den erfindungsgemässen Kamillenextrakten beträgt mindestens 0,4, vorzugsweise 0,425—0,8 Gewichtsprozent, bezogen auf die eingesetzte Drogenmenge mit einem Flügelblattanteil von 5—30 Gewichtsprozent.

Der Gehalt an Anzulen bei den erfindungsgemässen Extrakten ist beispielsweise 3 mg% vorzugsweise 3—15 mg%.

Beispiele
Beispiel 1

Ein Gemisch aus 320 g Kamillenblüten (ätherisches Öl 750 mg%; Anzulen 42 mg%) und 80 g Flügelblättern (ätherisches Öl 196 mg%; Azulen 0,4 mg%) wird mit 2100 g Ethanol (40 Gewichtsprozent) in einem Muldenmischer bei einer Drehzahl des Mischwerks von 65 Umdrehungen pro Minute extrahiert. Nach 90 Minuten wird das Drogengut abgepresst und der Extrakt filtriert. Im Extrakt wird der Wirkstoffgehalt in bekannter Weise ermittelt:

| | |
|---|---|
| Azulen | 10,4 mg% |
| ätherisches Öl | 98,6 mg% |
| Apigenin und Apigenin-glucoside (ber. als Apigenin) | 141,0 mg% |
| Extraktivstoffe | 6,85 % |

Vergleichsbeispiel (bekanntes Verfahren) 400 g Kamillenblüten (wie oben angegeben) werden mit 2100 g Ethanol (40 Gewichtsprozent) in einem Muldenmischer bei einer Drehzahl des Mischwerks von 65 Umdrehungen pro Minute extrahiert. Nach 90 Minuten wird das Drogengut abgepresst und der Extrakt filtriert. Im Extrakt wird der Wirkstoffgehalt in bekannter Weise ermittelt:

| | |
|---|---|
| Azulen | 11,2 mg% |
| ätherisches Öl | 101,3 mg% |
| Apigenin und Apigenin-glucoside (ber. als Apigenin) | 65,3 mg% |
| Extraktivstoffe | 6,53% |

Beispiel 2

Ein Gemisch aus 240 g Kamillenblüten (ätherisches Öl 750 mg%; Azulen 42 mg%) und 60 g Flügelblättern (ätherisches Öl 196 mg%; Azulen, 0,4 mg%) wird mit 2100 g Propanol-(2) (33 Gewichtsprozent) in einem Muldenmischer bei 65 Umdrehungen pro Minute extrahiert. Nach 90 Minuten wird das Drogengut abgepresst und der Extrakt filtriert. Im Extrakt wird der Wirkstoffgehalt in bekannter Weise ermittelt:

| Azulen | 4,9 mg% |
| ätherisches Öl | 87,6 mg% |
| Apigenin und Apigenin-glucoside (ber. als Apigenin) | 106,4 mg% |
| Extraktivstoffe | 7,09 % |

Vergleichsbeispiel (bekanntes Verfahren) 300 g Kamillenblüten (wie oben angegeben) werden mit 2100 g Propanol-(2) (33 Gewichtsprozent) in einem Muldenmischer bei 65 Umdrehungen pro Minute extrahiert. Nach 90 Minuten wird das Drogengut abgepresst und der Extrakt filtriert. Im Extrakt wird der Wirkstoffgehalt in bakannter Weise ermittelt:

| Azulen | 6,7 mg% |
| ätherisches Öl | 82,1 mg% |
| Apigenin und Apigenin-glucoside (ber. als Apigenin) | 53,0 mg% |
| Extraktivstoffe | 6,6 % |

**Patentansprüche**

1. Kamillenextrakte mit erhöhtem Flavongehalt von mindestens 80 mg% an natürlichen Flavonen (bezogen auf den Extrakt), die durch Extraktion einer Kamillendroge und Kamillenflügelblättern erhalten werden.

2. Kamillenextrakt mit einem erhöhten Gehalt an natürlichen Flavonen, wobei dieser Flavongehalt von dem Zusatz von Flügelblättern vor oder während der Extraktion herrührt.

3. Verfahren zur Herstellung von flavonreichen Kamillenextrakten, dadurch gekennzeichnet, daß diese durch Extraktion einer Kamillendroge unter Zusatz vom Kamillenflügelblättern erhalten werden.

4. Verfahren zur Herstellung von flavonreichen Kamillenextrakten, dadurch gekennzeichnet daß diese durch Extraktion einer Kamillendroge unter Zusatz von Kamillenflügelblättern mit gegebenenfalls wasserhaltigen aliphatischen ein- oder mehrwertigen Alkoholen bei Temperaturen zwischen 10 bis 50°C erhalten werden.

5. Kamillenextrakte, erhältlich nach Anspruch 3 oder 4.

6. Verwendung von Kamillenflügelblättern bei der Extraktion einer Kamillendroge.

7. Verwendung von Kamillenflügelblättern als Zusatz zu einer Kamillendroge und gegebenenfalls anschliessende Extraktion des aus der Kamillendroge und den Flügelblättern erhaltenen Produktes.

**Revendications**

1. Extraits de camomille ayant une teneur élevée en flavones, d'au moins 80 mg % de flavones naturelles (par rapport à l'extrait), qui sont obtenus par extraction d'une drogue de camomille et de fleurs ligulées de camomille.

2. Extrait de camomille ayant une teneur élevée en flavones naturelles, cette teneur en flavones provenant de l'addition de fleurs ligulées de camomille avant ou pendant l'extraction.

3. Procédé de préparation d'extraits de camomille riches en flavones, caractérisé en ce que ceux-ci sont obtenus par extraction d'une drogue de camomille avec addition de fleurs ligulées de camomille.

4. Procédé de préparation d'extraits de camomille riches en flavones, caractérisé en ce que ceux-ci sont obtenus par extraction d'une drogue de camomille avec addition de fleurs ligulées de camomille, par des mono- ou polyalcools aliphatiques contenant éventuellement de l'eau, à des températures comprises entre 10 et 50°C.

5. Extraits de camomille, obtenus selon la revendication 3 ou 4.

6. Utilisation de fleurs ligulées de camomille dans l'extraction d'une drogue de camomille.

7. Utilisation de fleurs ligulées de camomille comme additif à une drogue de camomille, cette addition étant éventuellement suivie d'une extraction du produit obtenu à partir de la drogue de camomille et des fleurs rigulées.

**Claims**

1. Camomile extracts having an increased flavone content of at least 80 mg-% natural flavones (based on the extract), which are obtained by extraction of a camomile drug and camomile wing petals.

2. A camomile extract having an increased content of natural flavones, this flavone content emanating from the addition of wing petals before or during the extraction.

3. A process for the production of camomile extracts rich in flavones, characterized in that they are obtained by extraction of a camomile drug with addition of camomile wing petals.

4. A process for the production of camomile extracts rich in flavones, characterized in that they are obtained by extraction of a camomile drug with addition of camomile wing petals with optionally aqueous aliphatic, mono- or polyhydric alcohols at temperatures of 10 to 50°C.

5. Camomile extracts obtainable by the process claimed in claim 3 or 4.

6. The use of camomile wing petals in the extraction of a camomile drug.

7. The use of camomile wing petals as an additive to a camomile drug and, optionally, subsequent extraction of the product obtained from the camomile drug and the wing petals.